# EUROPEAN PATENT SPECIFICATION

(11) **EP 3 320 834 B1**
(45) Date of publication and mention of the grant of the patent: **07.06.2023**
(21) Application number: 16839014.4
(22) Date of filing: 29.07.2016
(51) Int. Cl.: A61B 5/02, A61B 5/022, A61B 5/00

(54) **PULSE WAVE MEASUREMENT DEVICE**
PULSWELLENMESSVORRICHTUNG
DISPOSITIF DE MESURE D'ONDE DE POULS

(30) Priority: 24.08.2015 JP 2015164696
(43) Date of publication of application: 16.05.2018
(73) Proprietor: Omron Healthcare Co., Ltd., Muko-shi, Kyoto 617-0002 (JP)
(72) Inventor: KATO, Yuki, Muko-shi Kyoto 617-0002 (JP); SHIGIHARA, Noriko, Muko-shi Kyoto 617-0002 (JP); WAKAMIYA, Masayuki, Muko-shi Kyoto 617-0002 (JP); OGURA, Toshihiko, Muko-shi Kyoto 617-0002 (JP)
(74) Representative: Isarpatent
(86) International application number: PCT/JP2016/072354
(87) International publication number: WO 2017/033667

(56) References cited:
- JP-A- H0 467 839
- JP-A- H03 186 247
- JP-A- S57 103 628
- JP-A- 2004 188 183
- JP-A- 2008 168 032
- JP-A- 2015 144 628
- US-A- 5 179 956
- US-A1- 2006 047 207

## Description

### Technical Field

The present invention relates to a pulse wave measurement device.

### Background Art

Usually, a press type pressure measurement device is known which measures a contact pressure against an object to be measured, while applying a pressure against the object to be measured. As a device to which such a press type pressure measurement device is applied, there is a pulse wave measurement device.

A pulse wave measurement device is a device which, in order to measure a pressure pulse wave that is generated in an artery located in a position which is relatively shallow from the skin of a living body, measures the pressure pulse wave while pressing a substrate having a pressure-sensitive element against the body surface. In order to know the health condition of a subject, it is very important to measure the pressure pulse wave of the subject by using such a pulse wave measurement device.

In such a press type pressure measurement device, it is usual to employ a sensor chip which uses a distortion gauge and a diaphragm, as a pressure-sensitive element. As literatures on this kind of press type pressure measurement device, for example, there are Patent Literatures 1 to 4.

Patent Literature 1 discloses a pulse wave detection device in which a sensor chip in which a diaphragm is formed in a planar semiconductor substrate is mounted. The sensor chip has a configuration where the surface of a pressure-sensitive element is protected by a silicone rubber, and a portion which is of the surface of the silicone rubber, and which is to be in contact with a living body is covered by an electrically conductive rubber.

Patent Literature 2 discloses a contact pressure sensor including a semiconductor chip having opposite surfaces, at least one pressure sensing element provided in one of the opposite surfaces of the chip, a spacer member supporting the other surface of the chip, a substrate having a surface to which the spacer member is fixed, the one surface of the chip being pressed against an object which produces a pressure.

Patent Literature 3 discloses a pulse wave measuring apparatus including a semiconductor substrate having a pressure-sensing device on a main surface and a protection member with an accommodation space therein for accommodating the semiconductor substrate. Patent Literature 4 discloses a semiconductor substrate in which a pressure sensitive element is formed on one face, wherein, in pressing one face of the substrate against the skin of a living body, a pressure pulse wave generated from an artery positioned under the skin is detected.

### Citation List

### Patent Literatures

Patent Literature 1: JP 04 67839 A
Patent Literature 2: US 5 179 956 A
Patent Literature 3: US 2006/047207 A1
Patent Literature 4: JP H03 186247 A

### Summary of Invention

### Technical Problem

In the sensor chip disclosed in Patent Literature 1, the electrically conductive rubber exists in the surface which is to be in contact with a living body, and therefore static electricity due to friction with the skin in repeated uses can be suppressed from being generated. In the configuration of the sensor chip, the silicone rubber and the electrically conductive rubber overlap with each other above the pressure-sensitive element. Therefore, the thickness in the direction perpendicular to the pressure-sensitive surface is increased. When the thickness is increased as described above, there is a possibility that the accuracy of detecting a pressure pulse wave may be affected.

The invention has been conducted in view of the above circumstances. It is an object of the invention to provide a pulse wave measurement device in which, while reducing the thickness in a pressing direction, an influence of static electricity due to friction with the skin in repeated uses can be eliminated, and the accuracy of detecting a pressure pulse wave can be improved.

### Solution to Problem

The scope of the present invention is defined in the claims. The pulse wave measurement device of the invention includes: a pressure pulse wave sensor which includes: a semiconductor substrate on which a pressure-sensitive element and a first terminal portion that is electrically connected to the pressure-sensitive element are formed; and a rigid substrate which includes a second terminal portion that is electrically connected to the first terminal portion, and to which the semiconductor substrate is fixed; a flexible substrate which includes a wiring that is electrically connected to the second terminal portion, and to which the rigid substrate of the pressure pulse wave sensor is fixed; and a protective layer which is made from an insulating material, and which covers and protects the semiconductor substrate of the pressure pulse wave sensor, wherein the flexible substrate includes: a connector which is connected to the wiring; and a terminal which is held at a potential equal to a potential of the semiconductor substrate, and which is outwardly exposed in a position different from the connector, the outwardly exposed terminal is in contact with the protective layer, and a pressure-sensitive surface of the semiconductor substrate on which the pressure-sensitive element is formed is pressed through the protective layer against a body surface of a living body.

### Advantageous Effects of Invention

According to the invention, it is possible to provide a pulse wave measurement device in which, while reducing the thickness in a pressing direction, an influence of static electricity due to friction with the skin in repeated uses can be eliminated, and the accuracy of detecting a pressure pulse wave can be improved.

### Brief Description of Drawings

[Fig. 1] Fig. 1 is a view showing the configuration of a blood pressure measurement device 100 illustrating an embodiment of the invention.
[Fig. 2] Fig. 2 is a plan schematic view of a flexible substrate 16 on which pressure pulse wave sensors 1 of Fig. 1 are mounted.
[Fig. 3] Fig. 3 is a sectional schematic view taken along line A-A of Fig. 2.
[Fig. 4] Fig. 4 is a perspective view showing the configuration of main portions as seeing the pressure pulse wave sensor 1 of Fig. 1 from a side which is to be in contact with the skin.
[Fig. 5] Fig. 5 is a sectional schematic view showing the configuration of the vicinity of an interface between a semiconductor substrate 10A and surface coating layer 15 of the pressure pulse wave sensor 1 of Fig. 1.

### Description of Embodiments

Hereinafter, an embodiment of the invention will be described with reference to the drawings.

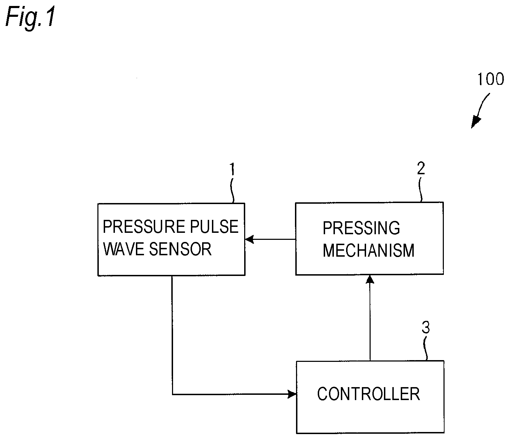

Fig. 1 is a block diagram showing the configuration of a blood pressure measurement device 100 which is a biological information measurement device illustrating an embodiment of the invention. For example, the blood pressure measurement device 100 is of the wrist wearing type which is used while attached to the wrist. The blood pressure measurement device 100 functions as a pulse wave measurement device which measures a pulse wave from a living body.

The blood pressure measurement device 100 includes: pressure pulse wave sensors 1; a pressing mechanism 2 for pressing the pressure pulse wave sensor 1 against the body surface of the subject; and a controller 3 which controls the pressing mechanism based on a signal output from the pressure pulse wave sensors 1, and which calculates biological information including the blood pressure of the subject, based on the signal.

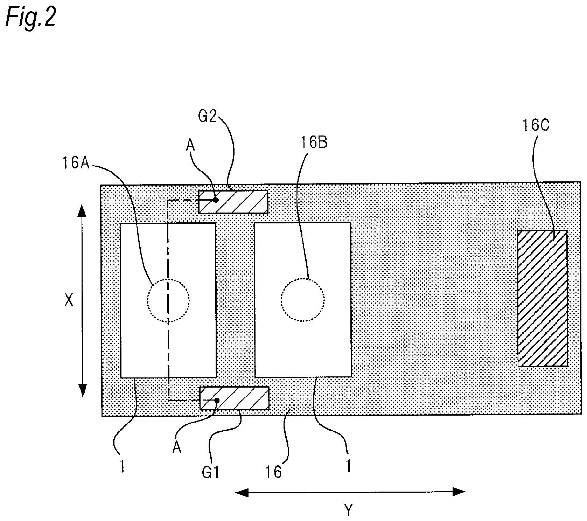

Fig. 2 is a plan view of a flexible substrate 16 on which the pressure pulse wave sensors 1 shown in Fig. 1 are mounted.

The flexible substrate 16 is formed into a rectangular shape in which a direction Y perpendicular to a direction X that is one direction coincides with the longitudinal direction. Two pressure pulse wave sensors 1 and a connector 16C are mounted on the surface of the substrate. The two pressure pulse wave sensors 1 are placed side by side in the direction Y.

In the flexible substrate 16, wirings connected respectively to connection terminals of the two pressure pulse wave sensors 1 are disposed in a resin film. The wirings are led to the connector 16C. The connector 16C is connected to a connector of a circuit board which is not shown, and on which the controller 3 of Fig. 1 and the like are formed.

Moreover, the flexible substrate 16 has through holes 16A, 16B in substantially middles of regions where the two pressure pulse wave sensors 1 are mounted, respectively.

In the flexible substrate 16, parts of the resin film covering the wirings are removed away, and, from portions where the parts are removed away, the outwardly exposed terminals G1, G2 are portions of exposed parts of the wirings that are to be grounded through the circuit board connected to the connector 16C.

The terminal G1 and the terminal G2 are placed in positions which are opposed to each other across a region between the two pressure pulse wave sensors 1, in the direction X.

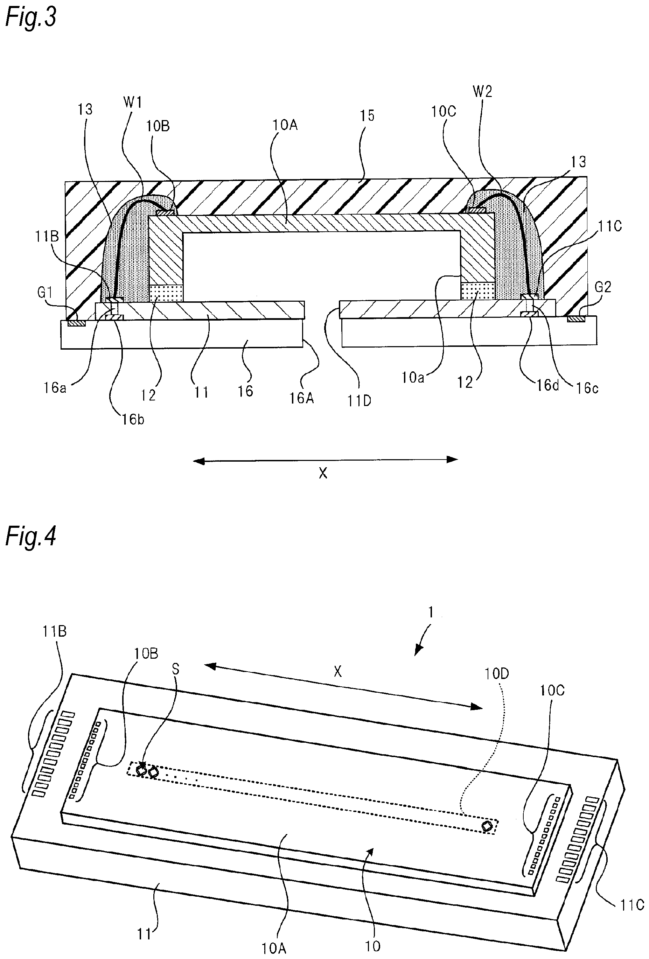

Fig. 3 is a sectional schematic view taken along line A-A shown in Fig. 2. Fig. 4 is a perspective view showing the configuration of main portions as seeing the pressure pulse wave sensor 1 from the side which is to be in contact with the skin. In Fig. 4, illustration of partial components is omitted. In Fig. 3, the components other than the flexible substrate 16 constitute the pressure pulse wave sensor 1.

As shown in Fig. 4, the pressure pulse wave sensor 1 includes a sensor chip 10, and a planar rigid substrate 11 to which the sensor chip 10 is fixed.

The sensor chip 10 includes a semiconductor substrate 10A made of a single crystal of silicon, that of a compound semiconductor such as gallium arsenide, or the like. The semiconductor substrate 10A is formed into a rectangular shape in which the direction X coincides with the longitudinal direction.

The rigid substrate 11 is made from a material which is sufficiently higher in rigidity than the semiconductor substrate 10A, such as a ceramic substrate, a glass substrate, or the like. The rigid substrate 11 is formed into a rectangular shape in which the direction X coincides with the longitudinal direction.

As shown in Fig. 4, a plurality of pressure-sensitive elements S each of which is configured by a bridge having four piezoresistance portions (distortion gauges, strain gauges), and which are used for sensing a contact pressure are arranged along the direction X on the surface (the surface on the side which is to be in contact with the skin of a living body) of the semiconductor substrate 10A. A pressure-sensitive element row 10D is configured by the plurality of pressure-sensitive elements S which are arranged in the direction X. In Fig. 3, illustration of the pressure-sensitive elements S is omitted.

In the semiconductor substrate 10A, in the surface opposite to the surface (hereinafter, referred to as the pressure-sensitive surface) on which the pressure-sensitive element row 10D is formed, as shown in Fig. 3, a concave portion 10a which is recessed in the direction (hereinafter, referred to as the sensor pressing direction) perpendicular to the pressure-sensitive surface is formed.

The concave portion 10a causes the semiconductor substrate 10A to have a configuration having a thin portion (diaphragm) in which the thickness in the sensor pressing direction is smaller than that of the other portion. Then, the pressure-sensitive element row 10D is formed in a region of the pressure-sensitive surface which is on the side opposite to the bottom surface of the concave portion 10a.

A portion which is of the surface opposite to the pressure-sensitive surface of the semiconductor substrate 10A, and which is other than the concave portion 10a (in other words, the surface in which the concave portion 10a is formed) is fixed to the surface of the rigid substrate 11 by an adhesive material 12. As the adhesive material 12, for example, a resin material of an ultraviolet curable resin is used.

The semiconductor substrate 10A is fixed to the surface of the rigid substrate 11 so that the concave portion 10a of the semiconductor substrate 10A communicates with the atmosphere only through a through hole 11D which is formed in the rigid substrate 11.

One pressure pulse wave sensor 1 of the two pressure pulse wave sensors 1 of the blood pressure measurement device 100 is mounted on the flexible substrate 16 so that the through hole 11D and the through hole 16A overlap with each other in a plan view as seen from the side of the pressure-sensitive surface. The other pressure pulse wave sensor 1 of the two pressure pulse wave sensors 1 of the blood pressure measurement device 100 is mounted on the flexible substrate 16 so that the through hole 11D and the through hole 16B overlap with each other in the plan view.

According to this configuration, a space which is in the pressure pulse wave sensor 1, and which is defined by the semiconductor substrate 10A, the adhesive material 12, and the rigid substrate 11 is kept at atmospheric pressure (reference pressure) by the through hole 11D of the rigid substrate 11, and the through hole 16A (or the through hole 16B) of the flexible substrate 16.

A first terminal portion 10B and first terminal portion 10C which are electrically connected to the pressure-sensitive element row 10D are placed in both end portions of the semiconductor substrate 10A in the direction X of the pressure-sensitive surface. Each of the first terminal portion 10B and the first terminal portion 10C is configured by a plurality of connection terminals which are arranged in a direction Y that is perpendicular to the direction X.

A second terminal portion 11B which is to be electrically connected to the first terminal portion 10B, and a second terminal portion 11C which is to be electrically connected to the first terminal portion 10C are disposed on the surface of the rigid substrate 11 to which the semiconductor substrate 10A is adhered and fixed.

In a plan view as seen in the direction perpendicular to the pressure-sensitive surface of the semiconductor substrate 10A, the second terminal portion 11B, the first terminal portion 10B, the first terminal portion 10C, and the second terminal portion 11C are arranged in this sequence along the direction X.

Each of the second terminal portion 11B and the second terminal portion 11C is configured by a plurality of connection terminals which are arranged in the direction Y that is perpendicular to the direction X. Each of the connection terminals of the second terminal portion 11B corresponds to one of the connection terminals of the first terminal portion 10B. Each of the connection terminals of the second terminal portion 11C corresponds to one of the connection terminals of the first terminal portion 10C.

As shown in Fig. 3, the connection terminals of the first terminal portion 10B are electrically connected to those of the second terminal portion 11B corresponding thereto by wires W1 which are first electroconductive members. Moreover, the connection terminals of the first terminal portion 10C are electrically connected to those of the second terminal portion 11C corresponding thereto by wires W2 which are second electroconductive members.

In the rigid substrate 11, a third terminal portion 16b configured by connection terminals which are connected to those of the second terminal portion 11B through wirings 16a, and a third terminal portion 16d configured by connection terminals which are connected to those of the second terminal portion 11C through wirings 16c are exposedly disposed on the surface opposite to the surface to which the semiconductor substrate 10A is fixed. The third terminal portions 16b, 16d are connected to wiring terminals which are exposed from the resin film of the flexible substrate 16.

The wiring terminals of the flexible substrate 16 include terminals which are to be connected to grounding terminals of the circuit board that is to be connected to the substrate through the connector 16C. Namely, the flexible substrate 16 includes wirings which are held at the ground potential, and exposed parts of the wirings are formed as the terminals G1, G2 in Fig. 2.

The peripheries of wires W1 and the wires W2 are separately covered and protected by protective members 13. A resin such as an epoxy or silicone resin is used as the protective members 13.

The semiconductor substrate 10A, the protective member 13 for the wires W1, the protective member 13 for the wires W2, and the rigid substrate 11 are covered by a surface coating layer 15 which is a protective layer for protecting the surface of the pressure pulse wave sensor 1. The surface coating layer 15 is made from an insulating material such as a silicone resin.

The surface coating layer 15 is formed also on the surface of the flexible substrate 16 on which the pressure pulse wave sensor 1 is mounted, so as to be in contact with at least parts of the terminals G1, G2 which are exposed from the flexible substrate 16. Preferably, the surface coating layer 15 may be formed so as to completely cover both the terminal G1 and the terminal G2.

The thus configured pressure pulse wave sensor 1 is used while the pressure-sensitive surface of the semiconductor substrate 10A on which the pressure-sensitive element row 10D is formed is pressed through the surface coating layer 15 against the body surface of a living body in a state where the pressure-sensitive element row 10D is located directly above an artery, and the direction X intersects (preferably, is perpendicular to) the traveling direction of the artery. From each of the pressure-sensitive elements S, therefore, an electric signal corresponding to distortion applied to the thin portion of the semiconductor substrate 10A, i.e., a signal indicating the pressure variation acting on the pressure-sensitive element S is output.

In the blood pressure measurement device 100, the controller 3 determines the optimum pressure-sensitive element and the optimum pressing force based on the signals output from the pressure pulse wave sensor 1, while adjusting the state of the pressurization which is applied on the body surface through the pressure pulse wave sensor 1 by the pressing mechanism 2. Thereafter, the controller measures the pressure pulse wave based on the signal which is output from the optimum pressure-sensitive element at the optimum pressing force, and calculates biological information such as the blood pressure value and the pulse rate based on the pressure pulse wave.

When the blood pressure measurement device 100 is continuously used in this way, the friction between the surface coating layer 15 and the body surface causes negative charges to be generated in the surface coating layer 15, and the negative charges are held in the interface between the surface coating layer 15 and the pressure-sensitive surface.

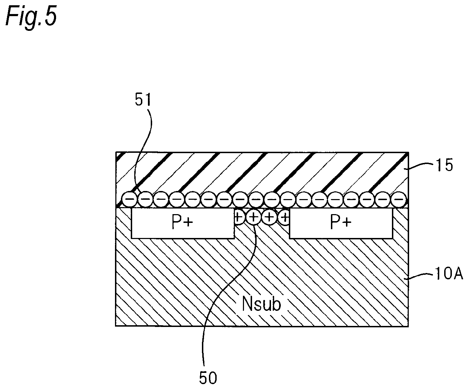

Fig. 5 is an enlarged sectional schematic view of the vicinity of the interface between the sensor chip 10 and the surface coating layer 15.

In the example of Fig. 5, the semiconductor substrate 10A of the sensor chip 10 is the N type, and piezoresistance portions are configured by P type semiconductor layers formed in the semiconductor substrate 10A.

The friction between the surface coating layer 15 and the body surface causes negative charges 51 to be generated in the surface coating layer 15, and the negative charges 51 are held in the interface between the surface coating layer 15 and the pressure-sensitive surface. When the negative charges 51 are held in the interface, an inversion channel is formed by positive charges 50 which are produced in a region between the piezoresistance portions (P+ regions) in the semiconductor substrate 10A. The inversion channel causes the offset voltage of the piezoresistance portions to be gradually raised.

In the blood pressure measurement device 100 of Fig. 1, the surface coating layer 15 is exposed from the flexible substrate 16, and in contact with the terminals G1, G2 which are held at the ground potential. Therefore, the negative charges which are generated in the surface coating layer 15 are discharged through the terminals G1, G2.

Even when negative charges are generated by friction, therefore, holding of the negative charges in the interface can be suppressed, and the offset voltage can be maintained constant. As a result, the accuracy of detecting a pressure pulse wave can be improved.

According to the blood pressure measurement device 100, moreover, the necessity of suppression of generation of static electricity due to friction is eliminated, and therefore the configuration where only the surface coating layer 15 is disposed on the pressure-sensitive surface can be employed. Consequently, the distance between the pressure-sensitive surface and the skin of a living body can be minimized, and the accuracy of detecting a pressure pulse wave can be improved.

In the rigid substrate 11, a configuration may be possible where the terminals G1, G2 are formed separately from the second terminal portion 11B and the second terminal portion 11C, and the surface coating layer 15 and the terminals G1, G2 are contacted with each other on the rigid substrate 11.

When the miniaturization of the pressure pulse wave sensor 1 is advanced, however, the space for forming the terminals G1, G2 cannot be sufficiently ensured on the rigid substrate 11. When the terminals G1, G2 are formed on the rigid substrate 11, wirings and terminals for holding the terminals G1, G2 at the ground potential must be added to the rigid substrate 11, and terminals which are to be connected to the terminals must be added to the flexible substrate 16, whereby the production cost is increased, and the design layout is complicated. In the configuration having two pressure pulse wave sensors 1, particularly, terminals must be additionally formed on each of the rigid substrates 11 of the two pressure pulse wave sensors 1, and therefore the cost increase becomes remarkable.

By contrast, the blood pressure measurement device 100 of the embodiment has the simple configuration where the grounding wires which are usually included in the flexible substrate 16 are exposed to be formed as the terminal G1, G2, and the terminal G1, G2 are covered by the surface coating layer 15. Therefore, the production cost is not increased, and the design layout is not complicated. Moreover, also the pressure pulse wave sensor 1 can be easily miniaturized.

In the blood pressure measurement device 100, moreover, the terminals G1, G2 are juxtaposed in the direction X across the region between the two pressure pulse wave sensors 1. Therefore, the surface coating layer 15 can be grounded in positions substantially equidistant from the pressure-sensitive surfaces of the two pressure pulse wave sensors 1, and the offset voltages of the two pressure pulse wave sensors 1 can be controlled in a substantially similar manner.

The presently disclosed embodiment should be considered in all respects to be illustrative and not restrictive. The scope of the invention is indicated by the appended claims rather than the foregoing description, and all changes which come within the meaning and range of equivalents thereof are intended to be embraced therein.

In the above, for example, the blood pressure measurement device of the wrist wearing type which detects the pressure pulse wave of the radial artery in the wrist has been described. Alternatively, a measurement device which is to be applied to the carotid artery or the dorsalis pedis artery may be possible.

The blood pressure measurement device 100 has the two pressure pulse wave sensors 1. When at least one pressure pulse wave sensor 1 is provided, the pressure pulse wave can be detected, and biological information can be measured.

The blood pressure measurement device 100 is configured so that the semiconductor substrate 10A is held at the ground potential, and the terminals G1, G2 are held at the ground potential. When the semiconductor substrate 10A and the surface coating layer 15 can be set to the same potential, it is possible to prevent negative charges from being held in the interface between the semiconductor substrate 10A and the surface coating layer 15. Therefore, the holding potential of the semiconductor substrate 10A may not be the ground potential. In a configuration where the semiconductor substrate 10A is held at a predetermined potential other than the ground, the terminals G1, G2 may be configured so as to be held at the predetermined potential.

As described above, the following matters are disclosed in the description.

The disclosed pulse wave measurement device includes: a pressure pulse wave sensor which includes: a semiconductor substrate on which a pressure-sensitive element and a first terminal portion that is electrically connected to the pressure-sensitive element are formed; and a rigid substrate which includes a second terminal portion that is electrically connected to the first terminal portion, and to which the semiconductor substrate is fixed, the pressure pulse wave sensor to be used while a pressure-sensitive surface on which the pressure-sensitive element is formed is pressed against a body surface of a living body; a flexible substrate which includes a wiring that is electrically connected to the second terminal portion, and to which the rigid substrate is fixed; and a protective layer which is made from an insulating material, and which covers and protects the semiconductor substrate of the pressure pulse wave sensor, the flexible substrate includes a terminal which is held at a potential equal to a potential of the semiconductor substrate, and which is outwardly exposed, and the terminal is in contact with the protective layer.

In the disclosed pulse wave measurement device, a pressure-sensitive element row configured by a plurality of pressure-sensitive elements which are arranged in one direction, and the first terminal portion that is electrically connected to the pressure-sensitive element row are formed on the semiconductor substrate of the pressure pulse wave sensor, the pressure pulse wave sensor is used while the pressure-sensitive surface on which the pressure-sensitive element row is formed is pressed through the protective layer against the body surface of the living body in a state where the one direction intersects a traveling direction of an artery of the living body, and the two pressure pulse wave sensors are fixed side by side to the flexible substrate in a direction perpendicular to the one direction, the flexible substrate includes the two outwardly exposed terminals, and the two outwardly exposed terminals are placed in positions which are opposed to each other across a region between the two pressure pulse wave sensors, in the one direction.

### Industrial Applicability

According to the invention, it is possible to provide a pulse wave measurement device in which, while reducing the thickness in a pressing direction, an influence of static electricity due to friction with the skin in repeated uses can be eliminated, and the accuracy of detecting a pressure pulse wave can be improved.

### Reference Signs List

- 100: blood pressure measurement device
- 1: pressure pulse wave sensor
- 10: sensor chip
- 10A: semiconductor substrate
- S: pressure-sensitive element
- 10D: pressure-sensitive element row
- 10B, 10C: first terminal portion
- 11: rigid substrate
- 11B, 11C: second terminal portion
- 16: flexible substrate
- G1, G2: terminal to be grounded

## Claims

1. A pulse wave measurement device comprising:
a pressure pulse wave sensor (1) which includes: a semiconductor substrate (10A) on which a pressure-sensitive element (S) and a first terminal portion (10B, 10C) that is electrically connected to the pressure-sensitive element (S) are formed; and a rigid substrate (11) which includes a second terminal portion (11B, 11C) that is electrically connected to the first terminal portion (10B, 10C), and to which the semiconductor substrate (10A) is fixed;
a flexible substrate (16) which includes a wiring (16a, 16c) that is electrically connected to the second terminal portion (11B, 11C), and to which the rigid substrate (11) of the pressure pulse wave sensor (1) is fixed; and
a protective layer (15) which is made from an insulating material, and which covers and protects the semiconductor substrate (10A) of the pressure pulse wave sensor (1), wherein
the flexible substrate (16) includes: a connector (16C) which is connected to the wiring (16a, 16c); and a terminal (G1, G2) which is held at a potential equal to a potential of the semiconductor substrate (10A), and which is outwardly exposed in a position different from the connector (16C),
the outwardly exposed terminal (G1, G2) is in contact with the protective layer (15), and
a pressure-sensitive surface of the semiconductor substrate (10A) on which the pressure-sensitive element (S) is formed is arranged to be pressed through the protective layer (15) against a body surface of a living body,
**characterized in that**
the outwardly exposed terminal (G1, G2) is a portion of an exposed part of the wiring (16a, 16c) connected to the connector (16C).

2. The pulse wave measurement device according to claim 1, wherein
a pressure-sensitive element row (10D) configured by a plurality of pressure-sensitive elements (S) which are arranged in one direction, and the first terminal portion (10B, 10C) that is electrically connected to the pressure-sensitive element row (10D) are formed on the semiconductor substrate (10A) of the pressure pulse wave sensor (1), the pressure-sensitive surface on which the pressure-sensitive element row (10D) is formed is arranged to be pressed through the protective layer (15) against the body surface of the living body in a state where the one direction intersects a traveling direction of an artery of the living body, and
the two pressure pulse wave sensors (1) are fixed side by side to the flexible substrate (16) in a direction perpendicular to the one direction, the flexible substrate (16) includes the two outwardly exposed terminals (G1, G2), and
the two outwardly exposed terminals (G1, G2) are placed in positions which are opposed to each other across a region between the two pressure pulse wave sensors (1), in the one direction.

3. The pulse wave measurement device according to claim 1 or 2, wherein
the protective layer (15) covers the semiconductor substrate (10A) of the pressure pulse wave sensor (1), the rigid substrate (11), and the outwardly exposed terminal (G1, G2) of the flexible substrate (16).

## Patentansprüche

1. Pulswellenmessvorrichtung, umfassend:
einen Druckpulswellensensor (1), der umfasst: ein Halbleitersubstrat (10A), auf dem ein druckempfindliches Element (S) und ein erster Anschlussabschnitt (10B, 10C), der elektrisch mit dem druckempfindlichen Element (S) verbunden ist, ausgebildet sind; und ein starres Substrat (11), das einen zweiten Anschlussabschnitt (11B, 11C) umfasst, der elektrisch mit dem ersten Anschlussabschnitt (10B, 10C) verbunden ist, und an dem das Halbleitersubstrat (10A) befestigt ist;
ein flexibles Substrat (16), das eine Verdrahtung (16a, 16c) umfasst, die elektrisch mit dem zweiten Anschlussabschnitt (11B, 11C) verbunden ist, und an dem das starre Substrat (11) des Druckpulswellensensors (1) befestigt ist; und
eine Schutzschicht (15), die aus einem isolierenden Material hergestellt ist und die das Halbleitersubstrat (10A) des Druckpulswellensensors (1) bedeckt und schützt, wobei
das flexible Substrat (16) umfasst: einen Verbinder (16C), der mit der Verdrahtung (16a, 16c) verbunden ist; und einen Anschluss (G1, G2), der auf einem Potential gehalten wird, das gleich einem Potential des Halbleitersubstrats (10A) ist, und der an einer anderen Position als der Verbinder (16C) nach außen frei liegt,
der nach außen frei liegende Anschluss (G1, G2) in Kontakt mit der Schutzschicht (15) steht und
eine druckempfindliche Fläche des Halbleitersubstrats (10A), auf der das druckempfindliche Element (S) ausgebildet ist, dafür ausgelegt ist, durch die Schutzschicht (15) hindurch gegen eine Körperoberfläche eines lebenden Körpers gedrückt zu werden,
**dadurch gekennzeichnet, dass**
der nach außen frei liegende Anschluss (G1, G2) ein Abschnitt eines frei liegenden Teils der Verdrahtung (16a, 16c) ist, der mit dem Verbinder (16C) verbunden ist.

2. Pulswellenmessvorrichtung nach Anspruch 1, wobei
eine Druckempfindliche-Elemente-Reihe (10D), die durch mehrere druckempfindliche Elemente (S) eingerichtet wird, die in einer Richtung angeordnet sind, und der erste Anschlussabschnitt (10B, 10C), der elektrisch mit der Druckempfindliche-Elemente-Reihe (10D) verbunden ist, auf dem Halbleitersubstrat (10A) des Druckimpulswellensensors (1) ausgebildet sind, wobei die druckempfindliche Fläche, auf der die Druckempfindliche-Elemente-Reihe (10D) ausgebildet ist, dafür ausgelegt ist, durch die Schutzschicht (15) hindurch gegen die Körperoberfläche des lebenden Körpers gepresst zu werden, während die eine Richtung eine Verlaufsrichtung einer Arterie des lebenden Körpers schneidet, und
die beiden Druckpulswellensensoren (1) nebeneinander an dem flexiblen Substrat (16) in einer Richtung senkrecht zu der einen Richtung befestigt sind, wobei das flexible Substrat (16) die beiden nach außen frei liegenden Anschlüsse (G1, G2) umfasst, und
die beiden nach außen frei liegenden Anschlüsse (G1, G2) an Positionen platziert sind, die über eine Region hinweg zwischen den beiden Druckpulswellensensoren (1) in der einen Richtung einander gegenüber liegen.

3. Pulswellenmessvorrichtung nach Anspruch 1 oder 2, wobei die Schutzschicht (15) das Halbleitersubstrat (10A) des Druckpulswellensensors (1), das starre Substrat (11) und den nach außen frei liegenden Anschluss (G1, G2) des flexiblen Substrats (16) bedeckt.

## Revendications

1. Dispositif de mesure d'onde de pouls comprenant :
un capteur d'onde de pulsation de pression (1) qui comporte : un substrat semi-conducteur (10A) sur lequel sont formés un élément sensible à la pression (S) et une première partie de borne (10B, 10C) qui est reliée électriquement à l'élément sensible à la pression (S) ; et
un substrat rigide (11) qui comporte une seconde partie de borne (11B, 11C) qui est reliée électriquement à la première partie de borne (10B, 10C), et auquel le substrat semi-conducteur (10A) est fixé ;
un substrat souple (16) qui comporte un câblage (16a, 16c) qui est relié électriquement à la seconde partie de borne (11B, 11C), et auquel le substrat rigide (11) du capteur d'onde de pulsation de pression (1) est fixé ; et
une couche de protection (15) qui est constituée d'un matériau isolant, et qui recouvre et protège le substrat semi-conducteur (10A) du capteur d'onde de pulsation de pression (1), dans lequel
le substrat souple (16) comporte : un connecteur (16C) qui est relié au câblage (16a, 16c) ; et une borne (G1, G2) qui est maintenue à un potentiel égal à un potentiel du substrat semi-conducteur (10A), et qui est exposée vers l'extérieur dans une position différente du connecteur (16C),
la borne exposée vers l'extérieur (G1, G2) est en contact avec la couche de protection (15), et
une surface sensible à la pression du substrat semi-conducteur (10A) sur lequel l'élément sensible à la pression (S) est formé est agencée pour être pressée à travers la couche de protection (15) contre une surface corporelle d'un corps vivant,
**caractérisé en ce que**
la borne exposée vers l'extérieur (G1, G2) est une partie d'une partie exposée du câblage (16a, 16c) reliée au connecteur (16C).

2. Dispositif de mesure d'onde de pouls selon la revendication 1, dans lequel
une rangée d'éléments sensibles à la pression (10D) configurée par une pluralité d'éléments sensibles à la pression (S) qui sont agencés dans une direction, et la première partie de borne (10B, 10C) qui est reliée électriquement à la rangée d'éléments sensibles à la pression (10D) sont formées sur le substrat semi-conducteur (10A) du capteur d'onde de pulsation de pression (1), la surface sensible à la pression sur laquelle la rangée d'éléments sensibles à la pression (10D) est formée est agencée pour être pressée à travers la couche de protection (15) contre la surface corporelle du corps vivant dans un état où ladite direction croise une direction de déplacement d'une artère du corps vivant, et
les deux capteurs d'onde de pulsation de pression (1) sont fixés côte à côte sur le substrat souple (16) dans une direction perpendiculaire à ladite direction, le substrat souple (16) comporte les deux bornes exposées vers l'extérieur (G1, G2), et
les deux bornes exposées vers l'extérieur (G1, G2) sont placées dans des positions qui sont opposées l'une à l'autre à travers une région entre les deux capteurs d'onde de pulsation de pression (1), dans ladite direction.

3. Dispositif de mesure d'onde de pouls selon la revendication 1 ou 2, dans lequel
la couche de protection (15) recouvre le substrat semi-conducteur (10A) du capteur d'onde de pulsation de pression (1), le substrat rigide (11) et les bornes exposées vers l'extérieur (G1, G2) du substrat souple (16).
